# EUROPÄISCHE PATENTSCHRIFT

(11) **EP 1 321 157 B1**
(45) Veröffentlichungstag und Bekanntmachung des Hinweises auf die Patenterteilung: **25.05.2005**
(21) Anmeldenummer: 02027091.4
(22) Anmeldetag: 03.12.2002
(51) Int. Cl.: A61M 5/32

(54) **Vorrichtung zur Entsorgung einer Kanüle und einer zugehörigen Schutzhülle**
Device for disposal of a cannula with its needle cap
Appareil pour jeter une cannule et son capuchon

(30) Priorität: 04.12.2001 DE 20119949 U
(43) Veröffentlichungstag der Anmeldung: 25.06.2003
(73) Patentinhaber: Steilner, Hansgeorg, Dr., 74354 Besigheim (DE)
(72) Erfinder: Suppan, Jakob, 74354 Besigheim (DE)
(74) Vertreter: Voth, Gerhard, Dipl.-Ing.

(56) Entgegenhaltungen:
- US-A- 5 356 385
- US-A- 5 482 207
- US-A- 5 791 471

## Beschreibung

Die Erfindung betrifft eine Vorrichtung zur Entsorgung einer Kanüle und einer zugehörigen Schutzhülle gemäß den Merkmalen des unabhängigen Anspruchs 1.

Aus der DE 196 45 514 ist ein Verfahren zum Entsorgen der bei einer Blutentnahmevorrichtung zur Blutentnahme mit einem Gefäß adaptierten Kanüle bekannt. Die Kanüle wird nach dem Abkoppeln des Gefäßes mit einem in einem Schutzgehäuse axial-beweglichen Zugelement gekoppelt und samt ihres Adapters vollständig in das Schutzgehäuse hineingezogen.

Das Problem bei der Entsorgung von gebrauchten Einwegkanülen liegt darin, dass diese Entsorgung bisher ein erhebliches Verletzungs- und Infektionsrisiko birgt. Während beim Aufbringen der Kanüle auf das Injektionsgerät die Kanüle noch durch eine Kappe geschützt ist, besteht bei dem Entfernen der gebrauchten und dann ungeschützten Kanüle die Gefahr der Stichverletzung. Zur sicheren Entsorgung wird häufig die Schutzkappe der Kanüle nach Gebrauch wieder aufgebracht. Insbesondere dieses Wiederaufbringen der Schutzkappe, das sogenannte Recapping ist aber sehr verletzungsträchtig. Wird das Recapping unterlassen, besteht Verletzungsgefahr aufgrund des Durchdringens der Wandung der Entsorgungsbehälters durch die gebrauchte Kanüle. Daher besteht die Forderung, dass Maßnahmen getroffen werden, die dieses Risiko vermeiden. Es muss möglich sein, dass der Anwender dieses Recapping einhändig betreiben kann.

Es ist weiter aus der DE 41 36 171 eine Versorgungs- und Entsorgungseinheit für Krankenhäuser bekannt. Diese besteht aus einem Träger, einem Tragegriff am Träger und mindestens einem am Träger lösbar angeordneten Abfallsammelbehälter. Der Abfallsammelbehälter ist ein Mehrwegbehälter, wobei mit der Versorgungs- und Entsorgungseinheit eine Möglichkeit gegeben werden soll, Kranke zu versorgen und die Entsorgung lückenlos, jedoch ohne Verletzungsgefahr durchzuführen.

Die US 5356385 beschreibt eine Vorrichtung zum Aufnehmen der Schutzhülle einer Nadel, wobei diese Schutzhülle durch 3 Backen, die elastisch gelagert sind gehalten wird. Nach Gebrauch der Nadel wird dieselbe wieder in die Schutzhülle zurückgesteckt. Durch einen Druck auf einen Schiebeschalter kann die Schutzhülle gelöst werden und das gesamte System, bestehend aus Flüssigkeitskammer, Nadel und Schutzhülle muss in geeigneten Vorrichtungen entsorgt werden.

Die US 5482207 beschreibt einen Behälter zum Aufnehmen von Kanülen, Hierzu wird eine Spritze in eine Öffnung des Behälters gesteckt. Durch einen motorischen Antrieb, der von einem Sensor geschaltet wird, erfolgt eine Entfernung der Kanüle.

Die US 5791471 beschreibt ein mechanisches System zum Halten einer Hülle für eine Kanüle und zum Aufnehmen der Kanüle in die Hülle und zum Entsorgen von gebrauchten Teilen.

Es hat sich gezeigt, dass die bekannten Systeme trotz aller Vorsichtsmaßnahmen ein gewisses Verletzungsrisiko in sich bergen. Dieses Verletzungsrisiko wird nur dann gemildert, wenn eine Entsorgung bzw. ein Recapping so durchgeführt werden kann, dass hierzu weder die Schutzhülle noch die Kanüle manuell gehandhabt werden muss. Solch ein System ist aus US 5 791 471 bekannt.

Aufgabe der vorliegenden Erfindung ist die Schaffung einer Vorrichtung zur Entsorgung einer Kanüle mit der zugehörigen Schutzhülle die einfach zu handhaben ist und die Verletzungsgefahr ausschließt.

Diese Aufgabe wird durch die Merkmale des unabhängigen Anspruchs 1 gelöst.

Der wesentliche Vorteil der Erfindung besteht darin, dass eine Vorrichtung zur Entsorgung einer Kanüle und einer zugehörigen Schutzhülle geschaffen wird, die wenige Bauteile aufweist und sicherstellt, dass die Entsorgung sehr einfach ist und zuverlässig funktioniert. Hierzu besteht die Vorrichtung aus wenigstens zwei Greifbacken, diese nehmen eine Schutzhülle auf. Die Schutzhülle kann manuell aus der Vorrichtung nicht mehr entnommen werden. Nach dem Gebrauch der Kanüle wird auch diese Kanüle in die Vorrichtung eingeführt und quasi wiederum mit der Schutzhülle versehen. Erst nachdem Schutzhülle und Kanüle wieder vereint sind, erfolgt durch ein Lösen der Greifbacken eine Freigabe der Schutzhülle und damit eine Zuführung von Schutzhülle und Kanüle in einen Entsorgungsbehälter.

Die Bewegung der Greifbacken erfolgt erfindungsgemäß mit einer drehbaren Abdeckung, diese Abdeckung greift über Mitnehmer in entsprechend gestaltete Nuten der Greifbacken ein, die Drehbewegung der Abdeckung wird in eine Längsbewegung der Greifbacken umgewandelt. Selbstverständlich besteht auch die Möglichkeit, die Greifbacken derart anzuordnen, dass diese sich sowohl radial als auch rotatorisch bewegen um die Schutzhülle mit der darin befindlichen Kanüle freizugeben.

Gemäß einer Ausgestaltung der Erfindung sind die Greifbacken auf einem Führungsschlitten angeordnet und weisen jeweils Nuten auf, in welche die Mitnehmer der Abdeckung eingreifen.

Eine weitere Ausgestaltung der Erfindung sieht vor, als Federelement für die Positionierung der Greifbacken einen O-Ring vorzusehen, welcher die Greifbacken umfasst. Es besteht auch die Möglichkeit, anstelle eines O- Rings eine Zugfeder vorzusehen, welche die beiden Greifbacken verbindet oder welche um die beiden Greifbacken geschlungen ist. Weiterbildungsgemäß weisen die Greifbacken im Aufnahmebereich für die Schutzhülle Mitnehmer zum verdrehsicheren Halten der Schutzhülle auf. Die handelsüblichen Schutzhüllen weisen üblicherweise an ihrem Unfang Stege auf, eine entsprechende Verzahnung an den Greifbacken bilden die Mitnehmer. Diese ver drehsichere Aufnahme hat den Vorteil, dass die Kanüle, welche ebenfalls verdrehsicher in die Schutzhülle einsteckbar ist, von dem Injektionsgerät abgeschraubt werden kann.

Eine weitere vorteilhafte Ausgestaltung des Gesamtsystems sieht vor, die Vorrichtung am Deckel eines Behälters anzuordnen und den Behälter zu verschweißen, sodass ein unbeabsichtigtes Öffnen nicht möglich ist.

Weiterbildungsgemäß ist eine Führungsplatte für die Greifbacken vorgesehen. Diese Führungsplatte stellt damit eine mögliche Verbindungsart zwischen Behälter und Greifbacken dar. Diese Führungsplatte weist sowohl Führungsnuten für die Greifbacken auf, außerdem ist sie mit Rast- Nasen versehen und wird an entsprechenden Rastvorrichtungen des Behälters befestigt.

Eine weitere Ausgestaltung der Erfindung sieht vor, die Abdeckung ebenfalls über eine Rastverbindung zu befestigen. Diese Abdeckung ist drehbar über die Rastverbindung an der Führungsplatte angeordnet.

Ein Verfahren zum Entsorgen von gebrauchten Einwegkanülen sieht vor, dass zunächst die Schutzhülle mit der beanspruchten Vorrichtung von dem Injektionsgerät abziehbar ist und zwar dadurch, dass das Injektionsgerät in die Öffnung der Vorrichtung eingesteckt wird. Die Schutzhülle wird von der Vorrichtung aufgenommen und das Injektionsgerät kann abgezogen werden. Nach dem injizieren, d.h. dem Gebrauch des Injektionsgerät wird dieses mit seiner Kanüle nochmals in die Öffnung der Vorrichtung eingesteckt, dabei gelangt die Kanüle wiederum in die Schutzhülle. Durch Drehen des Injektionsgeräts wird die Kanüle von diesem entfernt und verbleibt in der Schutzhülle. Damit Kanüle und Schutzhülle dem Behälter zugeführt werden kann, wird die Abdeckung derart gedreht, dass sich die Führungsbacken nach außen bewegen und die Schutzhülle freigeben. Die Rückstellung der Greifbacken erfolgt automatisch aufgrund der Federspannung des Federelements.

Diese und weitere Merkmale der Erfindung gehen nicht nur aus den Ansprüchen sondern auch aus der Beschreibung und den Zeichnungen hervor.

Die Erfindung wird nachfolgend anhand von Ausführungsbeispielen näher erläutert

Es zeigt:
- Fig. 1: in einer schematischen Darstellung eine Box zur sicheren Entsorgung für Kanülen von Injektionsgeräten,
- Fig. 2: eine Trägerplatte in Draufsicht und Seitenansicht,
- Fig. 3: zwei Greifbacken in verschiedenen Ansichten,
- Fig. 4: die Variante von Greifbacken ebenfalls in verschiedenen Ansichten,
- Fig. 5: eine Abdeckung in Draufsicht und Schnittdarstellung,
- Fig. 6 a-c: die Schnittdarstellung und die Draufsicht auf eine Entsorgungsbox,
- Fig. 7 a-c: die Schnittdarstellung und Draufsicht auf eine weitere Variante einer Entsorgungsbox.

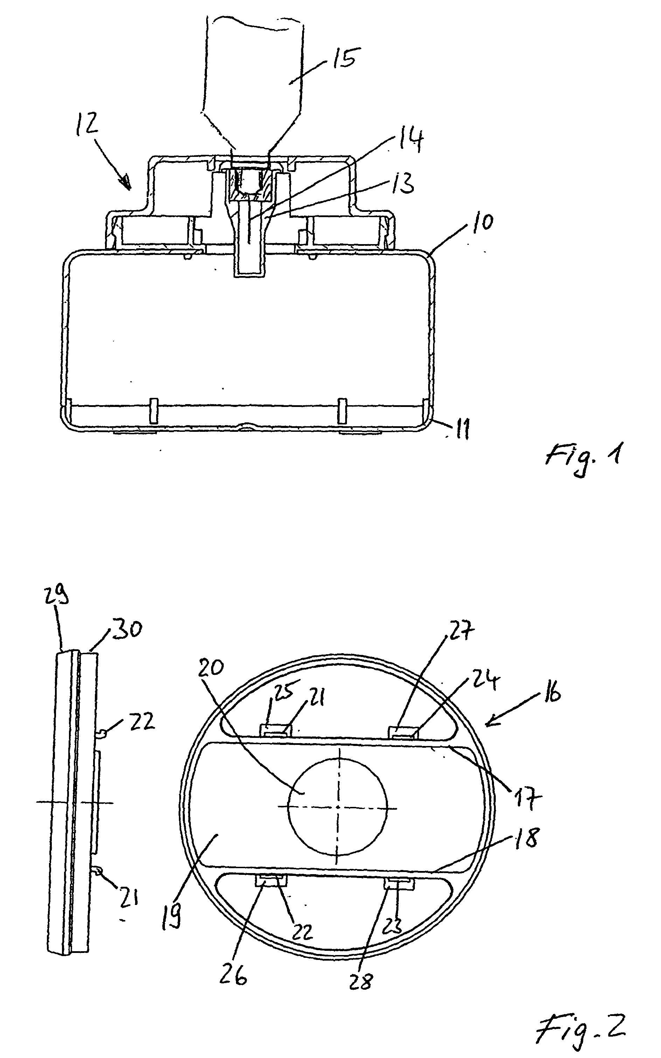

Die schematische Darstellung gemäß Fig.1 zeigt eine Box zur Entsorgung von Kanülen von Injektionsgeräten mit einem Gehäuse 10, das mit einem Boden 11 versehen ist. Der Boden 11 ist über eine Schweißverbindung mit dem Gehäuse 10 verbunden. Auf dem Gehäuse ist eine Halte- und Lösevorrichtung 12 für Kanülen und Schutzhüllen angeordnet. In dieser Halte- und Lösevorrichtung 12 befindet sich eine Schutzhülle 13 sowie eine darin fixierte Kanüle 14. Oberhalb dieser Schutzhülle und der Kanüle ist schematisch ein Injektionsgerät 15 gezeigt. Eine Drehbewegung des Injektionsgeräts bewirkt ein Lösen der Kanüle 14. Aus der Darstellung ist ersichtlich, dass die Kanüle ohne manuellen Eingriff und zuverlässig von dem Injektionsgerät entfernt werden kann.

Die weiteren Darstellungen sind geeignet die Funktionsweise und den Aufbau der Vorrichtung zum Entsorgen näher zu erläutern.

Fig. 2 zeigt eine Führungsplatte oder Trägerplatte 16 der Halte- und Lösevorrichtung 12 in Draufsicht und Seitenansicht. Diese Platte weist Führungsstege 17, 18 auf. Zwischen diesen Führungsstegen befindet sich eine Gleitebene 19. In der Mitte ist die Führungsplatte mit einer Durchtrittsöffnung 20 versehen. Außerdem besitzt die Führungsplatte wie auch in der Seitenansicht gezeigt, Rastnasen 21, 22, 23, 24. Diese Rastnasen greifen in hier nur schematisch angedeutete Öffnungen 25, 26, 27, 28 des in Fig. 1 dargestellten Gehäuses 10 und fixieren damit die Führungsplatte an diesem Gehäuse. Die Führungsplatte ist im wesentlichen konzentrisch ausgestaltet und weist umlaufend eine konische Fläche 29 auf, die bis zu einem Hinterschnitt 30 heranreicht. Diese konische Fläche und der Hinterschnitt dient zur Fixierung der weiter unten beschriebenen Abdeckung.

Fig. 3 zeigt zwei Greifbacken der Halte- und Lösevorrichtung 12 in verschiedenen Ansichten. Diese Greifbacken werden auf der in Fig. 2 dargestellten Führungsplatte angeordnet. Sie besitzen Führungsflächen 31, 32, welche sich in der Gleitebene 19 der Führungsplatte bewegen. Die Greifbacken 33, 34 besitzen im oberen Bereich Greifhaken 35, 36, welche segmentartig angeordnet sind und ein Greifen der Schutzhüllen bewirken. Die jeweils halbkreisförmige Öffnung 37, 38 ist wie in den beiden Außenansichten gezeigt, mit Einkerbungen 39, 40, 41 versehen, diese Einkerbungen sind auf Stege einer entsprechend aufzunehmenden Schutzhülle abgestimmt und bewirken eine verdrehsichere Fixierung einer darin aufzunehmenden Schutzhülle. Zur radialen Bewegung der Greifbacken dienen die Nuten 42, 43. Diese Nuten stehen mit später zu erläuternden Mitnehmern in Verbindung.

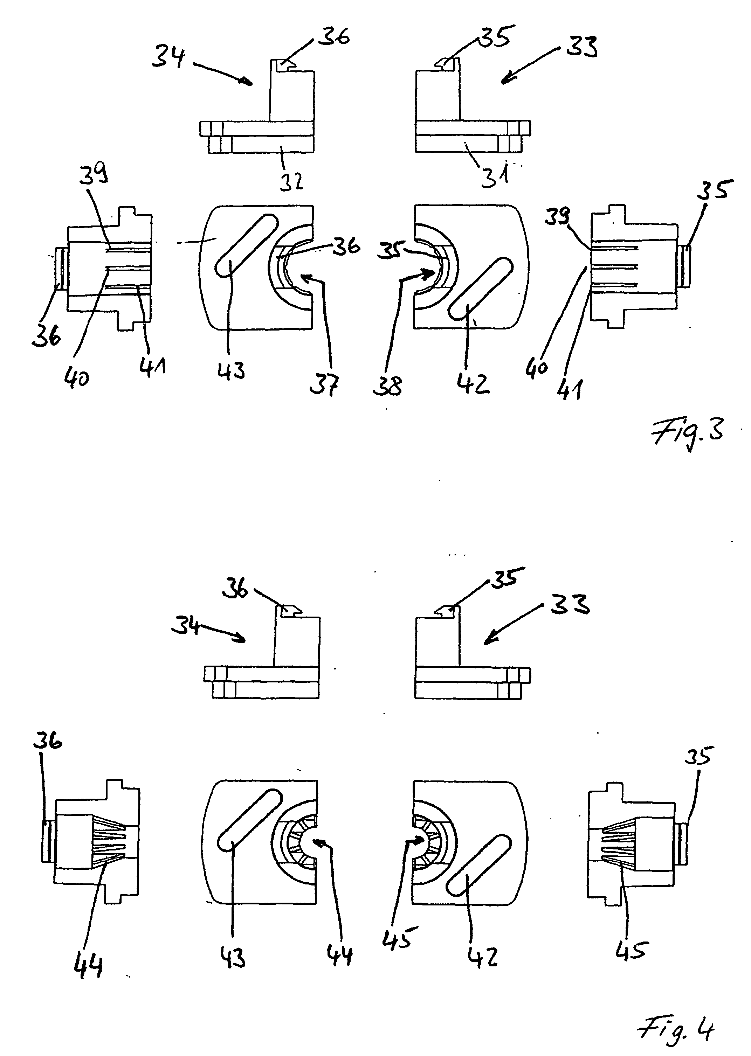

Fig. 4 zeigt eine Variante der in Fig. 3 gezeigten Greifbacken ebenfalls in unterschiedlichen Ansichten. Gleiche Bezugszeichen betreffen gleiche Teile. Die Variation besteht darin, dass die Aufnahme für Schutzhüllen eine etwas geänderte Gestaltung aufweist. Die Aufnahme zeigt einen konischen Flächenbereich 44, 45, welcher mit Einkerbungen versehen ist. Dieser Bereich ist an eine entsprechende Form von Schutzhüllen angepasst.

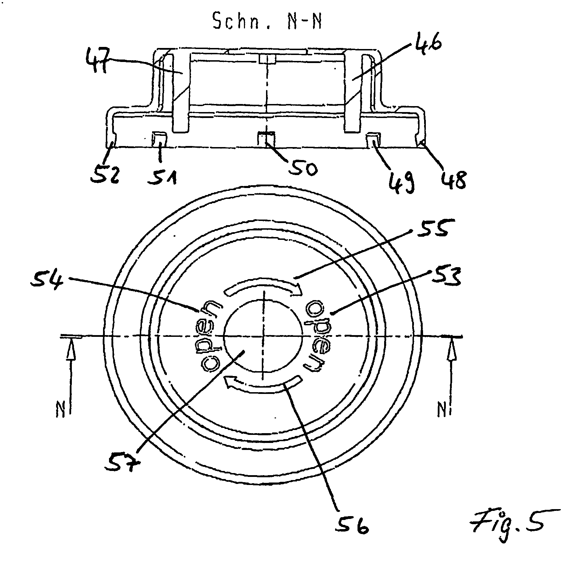

Fig. 5 zeigt eine Abdeckung für die in Fig. 4 und 3 gezeigten Greifbacken sowohl in Draufsicht als auch in Schnittdarstellung. Die Schnittdarstellung lässt zwei Mitnehmer 46, 47 erkennen. Diese Mitnehmer sind stiftförmig gestaltet und greifen in die in den Fig. 3 und 4 gezeigten Nuten 42 und 43 ein. Die Abdeckung besitzt umlaufend angeordnet Rastnasen 48, 49, 50, 51, 52. Diese Rastnasen bilden in Verbindung mit dem Hinterschnitt 30 der Führungsplatte 16 eine Verrastung und schaffen damit eine zuverlässige Verbindung der bereits beschriebenen Teile. Die Draufsicht auf die Abdeckung zeigt den Schriftzug 53, 54 und Pfeile 55, 56 und damit für die Bedienperson die Drehrichtung an, um ein Öffnen der Vorrichtung auszulösen. Der Deckel ist mit einer zentralen Bohrung 57 versehen, durch welche sowohl Schutzhülle als auch Kanüle eingesteckt werden können.

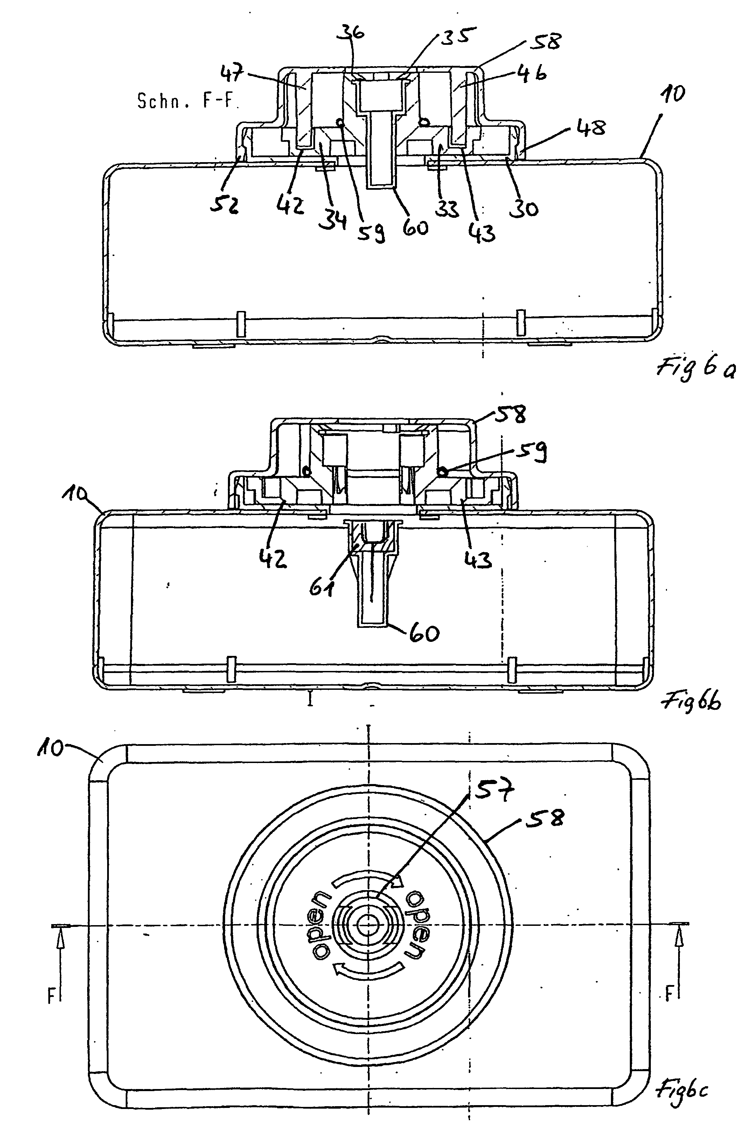

Die Fig. 6 a-c zeigen sowohl Schnittdarstellungen als auch eine Draufsicht auf eine Entsorgungsbox. In Fig. 6 a ist das gesamte montierte System der Vorrichtung zur Entsorgung von Kanüle und zugehöriger Schutzhülle erkennbar. Die Abdeckung 58 welche im Detail in Fig. 5 dargestellt ist, greift mit ihren Mitnehmern 46, 47 in die Nuten 42, 43 der Greifbacken 33, 34 ein. Die Greifbacken werden mittig gehalten durch einen O- Ring 59 der beide Greifbacken umschließt und das Federelement zum Zentrieren der Greifbacken darstellt. Die Abdeckung 58 greift mit den Rastnasen 48, 52 über den Hinterschnitt 30 und sorgt für eine zuverlässige Befestigung der Abdeckung 58 auf der Führungsplatte 16. Die Führungsplatte wiederum ist mit ihren in Fig. 2 beschriebenen Rastnasen 21-24 an dem Gehäuse 10 fixiert.

Wie in der Figur 6a dargestellt, befindet sich bereits eine Schutzhülle 60 in der Vorrichtung und wird durch die Greifhaken 35, 36 gehalten. In diese Schutzhülle kann nach dem Gebrauch die entsprechende und zugehörige Kanüle eingesteckt werden. Fig. 6 b zeigt, wie die Schutzhülle 60 mit der eingesteckten Kanüle 61 entsorgt wird. Hierzu ist es erforderlich, die Abdeckung 58 gemäß den in Fig. 6. c dargestellten Pfeilen zu drehen, dadurch werden die Greifbacken 33, 34 auseinandergezogen und die Schutzhülle mitsamt der Kanüle aufgrund der Schwerkraft in die Box befördert. Durch die Federwirkung des O-Rings 59 gehen anschließend die Greifbacken in die Ausgangsposition gemäß Fig. 6 a zurück.

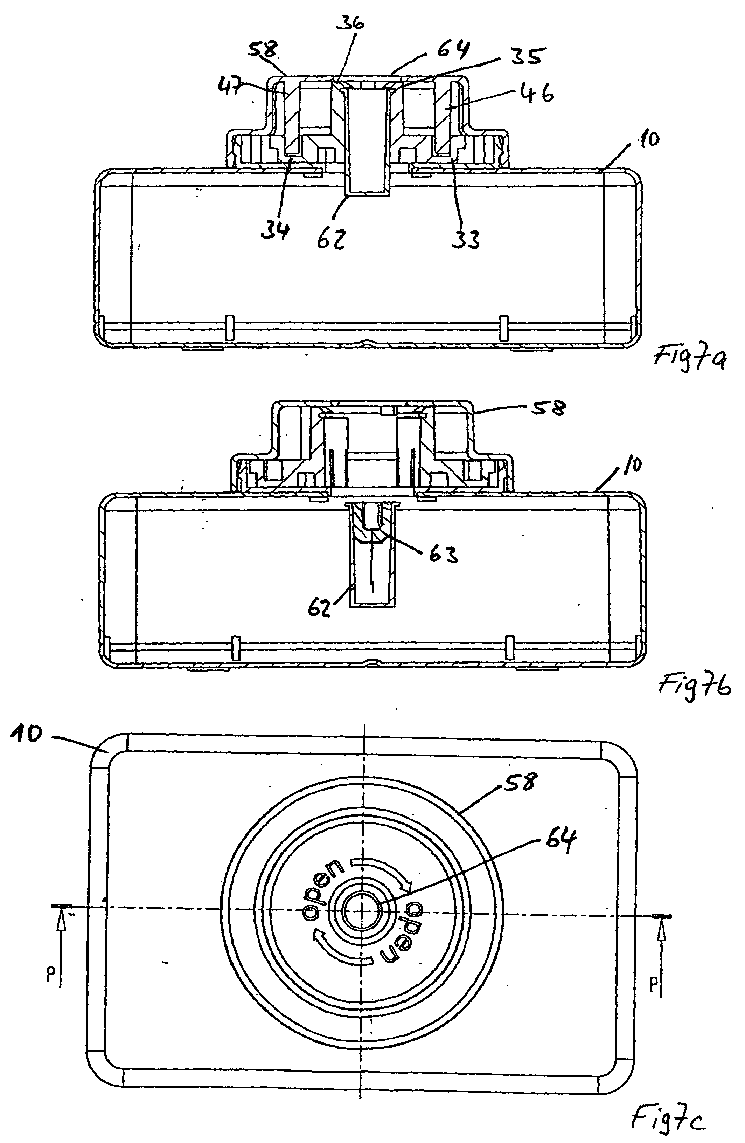

Die Fig. 7 a-c zeigen sowohl in Schnittdarstellungen als auch in einer Draufsicht eine Variante der Entsorgungsbox. Gleiche Teile sind mit gleichen Bezugszeichen versehen. Gemäß diesen Figuren sind lediglich die Greifbacken 33, 34 mit einem anderen Profil, und zwar mit einem Profil gemäß Fig. 3 ausgestattet, welches es erlaubt, Schutzhüllen 62 zu entsorgen, die die in den Figuren gezeigte Form aufweisen.

Abschließend wird nochmals das gesamte Verfahren zum Benutzen der Box zur Entsorgung von Kanülen von Injektionsgeräten beschrieben:

Zunächst wird von der Person, welche das Injektionsgerät bedient, eine ungebrauchte Kanüle mit ihrer äußeren Schutzhülle auf ein Injektionsgerät aufgesetzt. Die äußere Schutzhülle wird unter Ausübung eines leichten Druckes in die Öffnung 64 des Entsorgungsmechanismus gedrückt. Die Aufnahme und das Halten der Schutzhülle erfolgt durch die dort angeordneten Greifbacken. Die Person zieht anschließend das Injektionsgerät mit der darauf sich befindenden Injektionskanüle ab. Die Schutzhülle verbleibt in der Vorrichtung. Nach der Injektion wird die gebrauchte Einwegskanüle in die Schutzhülle zurückgesteckt ohne dass diese berührt werden muss. Durch ein Drehen des Injektionsgeräts löst sich die Kanüle vom Gewinde des Injektionsgeräts. Nach dem Entfernen des Injektionsgeräts bzw. der Kanüle wird durch eine Drehbewegung gemäß den auf der Box angebrachten Pfeilen die Greifbacken 33, 34 nach außen bewegt, so dass sich sowohl die Schutzhülle als auch die gebrauchte Einwegkanüle durch die Öffnung in die darunter befindliche Box bewegt. Beim Loslassen der Abdeckung 58 schieben sich die Greifbacken durch Hilfe des O- Rings in ihre ursprüngliche Stellung zurück.

Die Box ist derart bemessen, dass sie eine handelsübliche Packung beispielsweise 50 Stück von entsprechenden Kanülen aufnehmen kann. Sie ist handlich und kann dadurch zu jedem Einsatzort mitgenommen werden, wodurch unfallträchtige Entsorgungswege entfallen. Durch Haftfüße am Boden ist sie auch auf glatten Flächen standfest. An der Box können zu beiden Seiten des Verschlussmechanismus Piktogramme aufgebracht werden, die die Bedienungsschritte erläutern. Die gebrauchten Kanülen in der Box können besonders sicher entsorgt werden da sie aus der Box nicht mehr entnommen werden können und zusätzlich auch durch ihre Schutzkappe geschützt sind.

## Patentansprüche

1. Vorrichtung zur Entsorgung einer Kanüle (14) und der zugehörigen Schutzhülle (13) bestehend aus einer Aufnahmevorrichtung, welche die Schutzhülle (13) aufnimmt, wobei diese Aufnahmevorrichtung wenigstens zwei Greifbacken (33,34) aufweist, welche radial beweglich gelagert sind und eine Zentrierung der Greifbacken mit wenigstens einem Federelement (59) erfolgt, **dadurch gekennzeichnet, daß** eine drehbare Abdeckung (58) der Greifbacken (33,34) vorgesehen ist und diese Abdeckung (58) mit Mitnehmern (46, 47) ausgestattet ist, welche in Nuten (42,43) der Greifbacken (33,34) einwirken und eine Drehbewegung der Abdeckung (58) eine Bewegung der Greifbacken (33,34) derart bewirkt, dass eine von den Greifbacken (33,34) gehaltene Schutzhülle (13) und der Kanüle (14) einem Entsorgungsbehälter (11) zuführbar ist.

2. Vorrichtung nach Anspruch 1, **dadurch gekennzeichnet, dass** die Greifbacken (33,34) auf einem Führungsschlitten (17,18) angeordnet sind und jeweils eine Nut (42,43) aufweisen, wobei jede Nut mit jeweils einem Mitnehmer (46,47) der Abdeckung zusammenwirkt.

3. Vorrichtung nach Anspruch 1 oder 2, **dadurch gekennzeichnet, dass** das Federelement (59) ein die Greifbacken umfassender O-Ring ist.

4. Vorrichtung nach einem der vorigen Ansprüche, **dadurch gekennzeichnet, dass** die Greifbacken (33,34) im Aufnahmebereich für die Schutzhülle (13) Mitnehmer (39,40,41) zum verdrehsicheren Halten der Schutzhülle (13) aufweisen.

5. Vorrichtung nach einem der vorigen Ansprüche, **dadurch gekennzeichnet, dass** diese an dem Deckel (10) eines Behälters (11) angeordnet ist, wobei der Behälter mit dem Deckel verschweißt (10) ist.

6. Vorrichtung nach einem der vorigen Ansprüche, **dadurch gekennzeichnet, dass** die Führung der Greifbacken (33,34) über eine Führungsplatte (16) erfolgt, wobei die Führungsplatte (16) über eine Rastverbindung (30) mit dem Deckel des Behälters verbunden ist.

7. Vorrichtung nach Anspruch 6, **dadurch gekennzeichnet, dass** die Abdeckung (58) über eine Rastverbindung drehbar an der Führungsplatte (16) angeordnet ist.

8. Verfahren zum Entsorgen von gebrauchten Einweg- Kanülen, wobei die Kanülen (14) mit einer Schutzhülle (13) versehen sind und in einem ersten Verfahrensschritt die Schutzhülle (13) in die Öffnung einer Vorrichtung gemäß Anspruch 1 eingeführt wird, dort diese Schutzhülle (13) arretiert wird und von dem Injektionsgerät abziehbar ist und wobei nach dem Injizieren das Injektionsgerät mit der daran befindlichen Kanüle (14) in die Öffnung der Schutzhülle (13) eingesteckt und die Kanüle (14) durch Drehen des Injektionsgerätes von diesem gelöst wird und wobei nach dem Lösen der Kanüle (14) durch Drehen der Abdeckung (58) der Vorrichtung die darin festgehaltene Schutzhülle (13) und die Kanüle (14) in den Behälter (10,11) eingebracht werden.

9. Verfahren nach Anspruch 8, **dadurch gekennzeichnet, dass** nach dem Drehen der Abdeckung (58) und dem Entsorgen der benutzten Teile eine Rückstellung der Vorrichtung in die Ausgangsposition automatisch erfolgt und zur Aufnahme weiterer Einwegkanülen und deren Schutzhüllen bereit ist.

## Claims

1. Device for disposal of a cannula (14) and the needle cap (13) associated thereto, comprising a mounting device which accommodates the needle cap (13), wherein said mounting device has at least two gripping jaws (33, 34) which are supported and free to move in radial direction and which are centered by means of at least one spring element (59), **characterized in that** a rotatable cover (58) is provided for the gripping jaws (33, 34) and that said cover (58) is provided with tappets (46, 47) which will engage with slots (42, 43) located at the gripping jaws (33, 34) and that a rotation of the cover (58) will move the gripping jaws (33, 34) in such a way that a needle cap (13) held by the gripping jaws (33, 34) and the cannula (14) can be conveyed to a disposal box (11).

2. Device according to claim 1, **characterized in that** the gripping jaws (33, 34) are arranged on a guiding slide (17, 18) and each of the gripping jaws is provided with a slot (42, 43), wherein each slot cooperates with one tappet (46, 47) of the cover.

3. Device according to claims 1 and 2, **characterized in that** the spring element (59) is an O-ring surrounding the gripping jaws.

4. Device according to one of the preceding claims, **characterized in that** the gripping jaws (33, 34) are provided with tappets (39, 40, 41) in their needle cap (13) accommodating section in order to hold the needle cap (13) in a torsion-proof way.

5. Device according to one of the preceding claims, **characterized in that** said tappets are arranged at the cover (10) of the disposal box (11), wherein said box is welded together with said cover (10).

6. Device according to one of the preceding claims, **characterized in that** the gripping jaws (33, 34) are guided via a guiding plate (16), wherein said guiding plate (16) is connected to the cover of the box via a notch joint (30).

7. Device according to claim 6, **characterized in that** the cover (58) is rotatably arranged at the guiding plate (16) via a notch joint.

8. Method for the disposal of used one-way cannulas, wherein the cannulas (14) are provided with a needle cap (13) and in a first step of the method, said needle cap (13) is inserted in an opening of a device according to claim 1, where the needle cap (13) is clamped and can be released from the injection device, and wherein said injection device including the cannula (14) attached thereto is inserted in the opening of the needle cap (13) after injection and the cannula (14) is released therefrom by way of rotating the injection device; and wherein the needle cap (13) gripped by said device and the cannula (14) are conveyed to a disposal box (10, 11) by way of rotating the cover (58) of the device after having released the cannula (14).

9. Method according to claim 8, **characterized in that** the device will automatically reset and again be ready for receiving further one-way cannulas and their needle caps after the rotation of the cover (58) and the disposal of the used parts.

## Revendications

1. Dispositif pour l'élimination d'un cathéter (14) et de l'enveloppe de protection respectif (13), constitué d'un dispositif de logement, lequel accueille l'l'enveloppe de protection (13), tandis que ce dispositif de logement présente du moins deux mâchoires préhensiles (33,34), lesquelles sont appuyées mobiles radiales et un centrage des mâchoires préhensiles avec du moins un élément à ressort (59) a lieu, **caractérisé par** le fait de prévoir une couverture pivotante (58) des mâchoires préhensiles (33,34) et avec des entraîneurs (46,47), lesquels agissent dans des rainures (42,43) des mâchoires préhensiles (33,34) et qu'un mouvement rotatoire de la couverture (58) provoque un mouvement des mâchoires préhensiles (33,34) de façon qu'une enveloppe de protection (13) tenu des mâchoires préhensiles (33,34) (13) et le cathéter (14) est alimentable à un récipient de gestion des déchets (11).

2. Dispositif selon la revendication 1, **caractérisé par le fait que** les mâchoires préhensiles (33,34) sont arrangées sur un coulisseau de guidage (17,18) et chaque fois présentent une rainure (42,43), tandis que chaque rainure interagit avec chaque fois un entraîneur (46.47) de la couverture.

3. Dispositif selon la revendication 1 ou 2, **caractérisé par le fait que** l'élément à ressort (59) est un anneau à O qui comprend le mâchoires préhensiles.

4. Dispositif selon une des revendications précédentes, **caractérisé par le fait que** les mâchoires préhensiles (33,34) dans le secteur de logement pour l'enveloppe de protection (13) présentent des entraîneurs (39,40,41) pour tenir de façon résistante à la torsion l'enveloppe de protection (13).

5. Dispositif selon une des revendications précédentes, **caractérisé par le fait qu'**il est arrangé sur le couvercle (10) d'un récipient (11 ), le récipient étant soudé avec le couvercle (10).

6. Dispositif selon une des revendications précédentes, **caractérisé par le fait que** le guidage des mâchoires préhensiles (33,34) se fait moyennant une plaque de guidage (16), la plaque de guidage (16) étant relié au couvercle du récipient moyennant une liaison d'encoche (30).

7. Dispositif selon la revendication 6 **caractérisé par le fait que** la couverture (58) est appliquée à la plaque de guidage (16) de manière pivotante moyennant une liaison d'encoche.

8. Procédé pour éliminer des cathéters usés à jeter, dans lequel les cathéters (14) sont pourvus d'une enveloppe de protection (13) et dans une première phase du procédé l'enveloppe de protection (13) est introduite dans l'ouverture d'un dispositif selon la revendication 1, là cette enveloppe de protection (13) est bloquée et est retirable de l'appareil à injection et dans lequel après l'injection l'appareil à injection est enfilé avec le cathéter situé sur ce dernier (14) dans l'ouverture de l'enveloppe de protection (13) et le cathéter (14) moyennant la rotation de l'appareil à injection est détaché de ce dernier et dans lequel après le détachement du cathéter (14) moyennant la rotation de la couverture (58) du dispositif l'enveloppe de protection bloquée là-dedans (13) et le cathéter (14) sont introduits dans le récipient (10,11).

9. Procédé selon la revendication 8, **caractérisé par le fait qu'**après la rotation de la couverture (58) et l'élimination des parties usées une remise du dispositif dans la position initiale a lieu automatiquement et qu'il est prêt pour la réception d'ultérieurs cathéters à usage unique et de leurs enveloppes de protection.
